# EUROPEAN PATENT APPLICATION

(11) **EP 1 029 924 A1**
(43) Date of publication of application: **23.08.2000**
(21) Application number: 99938579.2
(22) Date of filing: 23.08.1999
(51) Int. Cl.: C12N 15/82, A01H 5/00

(54) **NUCLEIC ACIDS HAVING PROMOTER ACTIVITY AND TRANSGENIC PLANTS CONTAINING THE SAME**

(30) Priority: 24.08.1998 JP 25328298
(71) Applicant: Japan Tobacco Inc., Tokyo 105-8422 (JP)
(72) Inventor: SUZUKI, Shoichi, Japan Tobacco Inc., Toyoda-cho, Iwata-gun, Sizuoka 438-0802 (JP)
(74) Representative: Ruffles, Graham Keith
(86) International application number: JP9904519
(87) International publication number: WO0011197

(57) **Abstract**

Novel nucleic acids being capable of exerting a promoter activity in the phloem tissue and transgenic plants containing these nucleic acids and being capable of expressing a desired structural gene under the control of the nucleic acids. The above nucleic acids involve those having the base sequences represented by SEQ ID NOS:1 and 2 in Sequence Listing and those derived therefrom by substitution, deletion, insertion or addition of one or more bases and being capable of showing the promoter activity in plants and parts of these nucleic acids being capable of showing the promoter activity. The above transgenic plants are those which have been transformed by such a nucleic acid and a desired structural gene to be expressed in the plants functionally ligated to the downstream of the nucleic acid serving as a promoter thereof and can express the structural gene in the phloem tissue.

## Description

### TECHNICAL FIELD

The present invention relates to a nucleic acid which exerts promoter activity in the phloem tissue of a plant, and to a transformed plant that contains the nucleic acid and that can express a desired foreign gene in the phloem tissue thereof.

### BACKGROUND ART

It is known that phosphoenolpyruvate carboxykinase (PCK; EC4.1.1.49) is localized in the cytoplasms of bundle sheath cells and functions as an enzyme indispensable to C4 photosynthesis in the C4 plants of the type which utilizes the enzyme as the main decarboxylase (PCK type C4 plants). It is also known that the enzyme functions as a decarboxylase for C4 organic acids in CAM plants of the type which utilizes the enzyme as a decarboxylase (PCK type CAM plants). In C3 plants, a PCK relating to gluconeogenesis in germination of seeds is known, and in an algae, a PCK relating to concentration of carbon dioxide is known.

As for plant PCK genes, although cDNAs have been isolated from cucumber (C3 plant) (Dae-jae Kim et al., Plant Molecular Biology 26: 423-434, 1994) and *Urochloa panicoides* (Patrick M. Finnegan et al., Plant Molecular Biology 27: 365-376, 1995), respectively, the promoter regions thereof have not been reported. Further, none of these genes have been reported for their promoter activities in plants.

On the other hand, some promoters controlling the phloem-specific expression in plants have been reported (U.S. Patent No. 5,495,007, German Patent No. 4,306,832, International Publication Nos. WO 9304177, WO 9222582, and WO 9109050). However, none of these are the promoter of PCK gene.

As the transformation technology develops, it is becomingly possible to create transformed plants into which exogenous characters are introduced. In cases where it is necessary to express an introduced gene tissue (cell)-specifically, it is necessary to use a promoter controlling the tissue (cell)-specific expression. For example, since transportation and translocation of metabolites and the like are carried out trough sieve tubes, it is desired to express a gene which is introduced for the control of the translocation specifically in the vicinity of the sieve tubes. It is thought that isolation of a promoter gene which is strongly expressed in the vicinity of sieve tubes would be an effective tool for construction of transformed plants.

### DISCLOSURE OF THE INVENTION

Accordingly, an object of the present invention is to provide a novel nucleic acid which exerts promoter activity in the phloem tissue of a plant, and a transformed plant that contains the nucleic acid and that can express a desired foreign gene in the phloem tissue thereof.

As a result of intensive study, the present inventor has succeeded, for the first time, in identifying the promoter of the PCK gene of *Urochloa panicoides* which is a C4 plant, and in sequencing the promoter. Further, the present inventor succeeded in actually preparing plants transformed with the promoter and the desired structural genes ligated to a site downstream of the promoter, thereby completing the present invention.

That is, the present invention provides a nucleic acid having the nucleotide sequence shown in SEQ ID NO: 1 or 2 in SEQUENCE LISTING or a nucleic acid having a nucleotide sequence which is the same as the nucleotide sequence shown in SEQ ID NO: 1 or 2 in SEQUENCE LISTING except that one or a plurality of nucleotides are substituted, deleted, inserted or added, the latter nucleic acid having a promoter activity in phloem tissue of a plant, or a nucleic acid which is a part of anyone of the nucleic acids, that has a promoter activity in phloem tissue of a plant Further, the present invention provides a transformed plant transformed with the nucleic acid according to the present invention and a desired structural gene which is functionally ligated to a site downstream of the nucleic acid and which is controlled by the nucleic acid as a promoter, which transformed plant expresses the structural gene in its phloem tissue.

By the present invention, a novel nucleic acid which exerts promoter activity in the phloem tissue of a plant was provided. By using the nucleic acid of the present invention as a promoter, it was made possible to strongly express a target gene specifically in the vicinity of sieve tubes of a plant such as rice. The nucleic acid is also effective when it is desired to express the target gene also in glume, pollen and anther. The promoter having such a property is useful for expressing an insecticidal protein, anti-viral protein, disease resistant protein or the like, thereby giving disease resistance or insect resistance to plants. The promoter is also useful for expressing a protein (e.g., proteins regarding carbon metabolism and sugar transporters) for supplying a nutrient such as carbon source (saccharide) or nitrogen source from the source to the sink, or for expressing a protein for supplying a signal transducer from the sink to the source. Besides, the promoter is especially useful in cases where it is necessary to express a protein for the purpose of transporting a substance through sieve tubes.

### BRIEF DESCRIPTION OF THE DRAWINGS

Fig. 1 shows the structures of recombinant vectors pSB4PCK1 and pSB4PCK3 constructed in Examples, and shows the method for constructing these recombinant vectors.
Fig. 2 shows the structures of recombinant vectors pSB4PCK1bar and pSB4PCK3bar constructed in Examples, and shows the method for constructing these recombinant vectors.

### BEST MODE FOR CARRYING OUT THE INVENTION

By the method detailed in the Examples described below, the present inventor cloned two types of DNAs containing the promoter sequences of PCK genes of *Urochloa panicoides* and determined the nucleotide sequences thereof. The determined sequences are shown in SEQ ID NOs: 1 and 2 in SEQUENCE LISTING. The nucleic acids having these nucleotide sequences exert promoter activities specifically in the phloem tissue of plants (as described in Examples below, the nucleic acids exert promoter activities also in glume, pollen and anther).

In general, it is well-known in the art that there are cases wherein the physiological activity of a physiologically active nucleic acid is retained even if the nucleotide sequence of the nucleic acid is modified such that one or more nucleotides are substituted, deleted, inserted or added. Therefore, nucleic acids having the same nucleotide sequence as the SEQ ID NO: 1 or 2 except that the nucleotide sequences of the nucleic acids are modified such that one or more nucleotides are substituted, deleted, inserted or added, which have the promoter activities exerted in the phloem tissues of plants, are included within the scope of the present invention. It is preferred that the nucleotide sequences of these nucleic acids have homologies of not less than 70%, more preferably not less than 90%, still more preferably not less than 95%, with the nucleotide sequence shown in SEQ ID NO: 1 or 2. Further, it is preferred that these nucleic acids hybridize with the nucleic acid having the nucleotide sequence shown in SEQ ID NO: 1 or 2 under stringent conditions (i.e., hybridization is performed at 60 to 65°C using a common hybridization solution such as 5 x Denhardt's reagent 6 x SSC, 0.5% SDS).

Further, since it is known that promoter activities are exerted by nucleic acids with sizes of about 80 bp, pans, especially those with sizes of not less than about 80 bp, of the nucleic acid having the nucleotide sequence shown in SEQ ID NO: 1 or 2 in SEQUENCE LISTING or of a nucleic acid having a nucleotide sequence which is the same as the nucleotide sequence shown in SEQ ID NO: 1 or 2 in SEQUENCE LISTING except that one or a plurality of nucleotides are substituted, deleted, inserted or added, the latter nucleic acid having a promoter activity in phloem tissue of a plant are included in the scope of the present invention.

Since the origin and the nucleotide sequence of the nucleic acids according to the present invention have been clarified, the nucleic acids according to the present invention may easily be prepared or isolated by PCR using the genomic DNA of *Urochloa panicoides* as a template. In the present specification, the term "isolation" means cutting out the nucleic acid fragment from the genome or the like so as to enable the nucleic acid fragment to be cloned, or amplifying the nucleic acid fragment by a nucleic acid-amplification method such as PCR, or synthesizing the nucleic acid fragment.

By transforming a plant with the nucleic acid having the promoter activity according to the present invention, and with a desired structural gene which is functionally ligated to a site downstream of the nucleic acid of the present invention and which is controlled by the nucleic acid as a promoter, the structural gene may be expressed in the phloem tissue of the plant. Methods of transformation of plants *per se* are well-known in the art. Examples of the methods include electroporation (Toriyama K. et al., 1988; Bio/Technol. 6:1072-1074, Shimamoto K. et al., 1989; Nature 338:274-276, Rhodes C. A. et al., 1989; Science 240:204-207), PEG Method (Zhang W. et al., 1988; Theor. Appl. Genet. 76:835-840, Datta S.K. et al., 1990; Bio/Technol. 8:736-740), particle gun method (Gordon-Kamm W. J. et al., 1990; Plant Cell 2:603-618, Fromm M.E. et al., 1990; Bio/Technol. 8:833-839, Christou P. et al., 1991; Bio/Technol. 9:957-962) and the like. Among the methods for transformation, the agroinfection method via *Agrobacterium* is preferred since the reproducibility and efficiency are high, and the method may be applied to plants for which a regeneration method from protoplasts have not been established. The agroinfection method *per se* is well-known in the art and described in, for example, Japanese Patent No. 2649287 and U.S. Patent No. 5,591,616, and vectors for agroinfection are commercially available. By incorporating the nucleic acid having the promoter activity according to the present invention and the desired structural gene to be expressed into such a commercially available vector, a recombinant vector according to the present invention may easily be prepared. In cases where the desired structural gene is large so that it is difficult to directly incorporate the structural gene into the vector, the recombinant vector may be prepared by incorporating the structural gene into a vector for *E. coli*, which vector has a region homologous to the vector for *Agrobacterium*, and then allowing homologous recombination between the vectors in *Agrobacterium* (Komari, T. et al., Plant J. (1996) 10:165-174). Introduction of a plasmid into *Agrobacterium* may be carried out by, for example, the well-known triple cross method (Ditta G. et al., 1980; Proc. Natl. Acad. Sci. USA 77:7347-7351; Komari, T et al., *supra*). In cases where the nucleic acid of the present invention having the promoter activity and the structural gene are incorporated into a vector for agroinfection, the nucleic acid and the structural gene downstream thereof are inserted between the right and left border sequences of the T-DNA. Further, in cases where a commercially available vector for *Agrobacterium* is used, the promoter region of the commercially available vector may be cut out and the nucleic acid having the promoter activity may be inserted into the site at which the original promoter region was located, as described in the Examples below. As the vector for *Agrobacterium*, the super binary vector (i.e., the vector containing the virulence region of the Ti plasmid pTiBo542 contained in *Agrobacterium tumefaciens* A281 and the right and left border sequences of the T-DNA of the Ti plasmid or Ri plasmid of a bacterium belonging to the genus *Agrobacterium*) described in Japanese Patent No. 2649287 may preferably be used. Transformation of plants by *Agrobacterium* into which the recombinant vector of the present invention was introduced may be carried out by the well-known methods such as the method described in Japanese Patent No. 2649287.

### Examples

The present invention will now be described in more concretely by way of examples thereof. However, the present invention is not restricted to the examples below.

### Example 1

### (1) Isolation of PCK Genomic Clone

DNAs were isolated from green leaves of *Urochloa panicoides* using phenol (Komari, T. Plant Science 60:223-229 (1989)). One hundred micrograms of the DNAs were subjected to partial digestion with a restriction enzyme Sau3AI (produced by Pharmacia). The resultant was concentrated by isopropanol precipitation, and the obtained concentrate was subjected to continuous density gradient centrifugation of NaCl with a concentration of 20% to 5% (W/V) (maximum 240,000 x g, 25°C, 4 hours and 30 minutes), followed by fractioning the resultant into small fractions. The size of the DNA in each fraction was determined by agarose gel electrophoresis and the DNAs from 23 to 15 kb was ligated to the BamHI site of lambda Dash II vector (trademark, produced by Stratagene) and a genomic library was prepared using Giga Pack Gold package mix (trademark, produced by Stratagene). Using the 880 bp region upstream of the KpnI site of PCK cDNA of *Urochloa panicoides* (Patrick M. Finnegan et al., *supra*) as a probe, the genomic library was screened by the conventional method (Sambrook,J., Fritsch, E.F. and Maniatis,T.: Molecular cloning: A laboratory manual, 2nd ed., Cold spring harbor laboratory press, Cold Spring Harbor NY (1989)) to obtain a plurality of positive clones. The clones were sequenced by T7 Sequencing TM Kit (trademark, Pharmacia) or by 373A DNA Sequencer (Applied Biosystems) to obtain PCK1 and PCK3 genes having very high homologies with the reported PCK cDNA. Since the homologies between the exon regions of the both genes and the cDNA were extremely high, both genes were thought to be PCK genes (Table 1). The 5' upstream region which is upstream of the translation initiation site of each gene was referred to as the promoter region. The determined nucleotide sequences of PCK1 and PCK3 are shown in SEQ ID NOs: 1 and 2, respectively.

**Table 1**

| Homology with PCK1 cDNA | | |
|---|---|---|
| PCK1 | EXON 2 | EXON 3 |
| | 100% (169/169) | 100% (179/179) |
| PCK3 | EXON 4 | EXON 6 |
| | 89.6% (138/154) | 93.0% (280/299) |

### (2) Construction of Vector for Checking PCK Promoter Activity

Using synthetic primers which were designed to give restriction sites at both ends and Takara EX Taq (trademark, Takara Shun, Co., Ltd.), the promoter regions of PCK1 and PCK3 were amplified by PCR method. The nucleotide sequences of the used synthetic primers were as follows:

### PCK1

- Forward Primer:: 5' CGCGTAAGCTTGATCCTGGAGCTTTATTATG 3'
- Reverse Primer:: 5' GATAGGATCCTCGTTCGAGCGTCCTGC 3'

### PCK3

- Forward Primer:: 5' GACGTAAGCTTGAGGAAGAGGTGGCTCTG 3'
- Reverse Primer:: 5' GATAGGATCCTCGTTCGAGCGTGTCCTGCA 3'

As for PCK3, since a fragment having a size shorter than the expected size was obtained, the entire nucleotide sequence was determined (SEQ ID NO: 3) using 373A DNA Sequencer (Applied Biosystems). As for PCK1, since a fragment having the expected size was obtained and since the sites cut with the restriction enzyme had the same nucleotide sequences as the both ends, respectively, it was judged that the desired fragment was obtained.

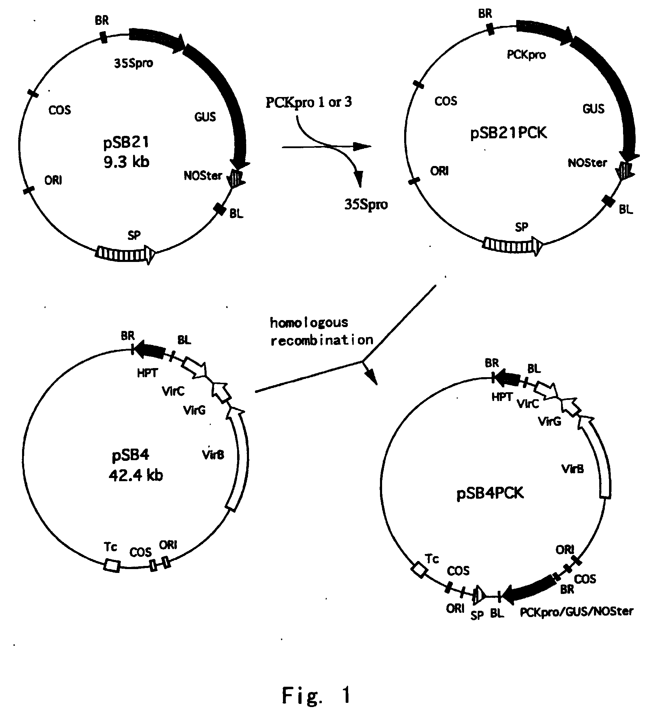

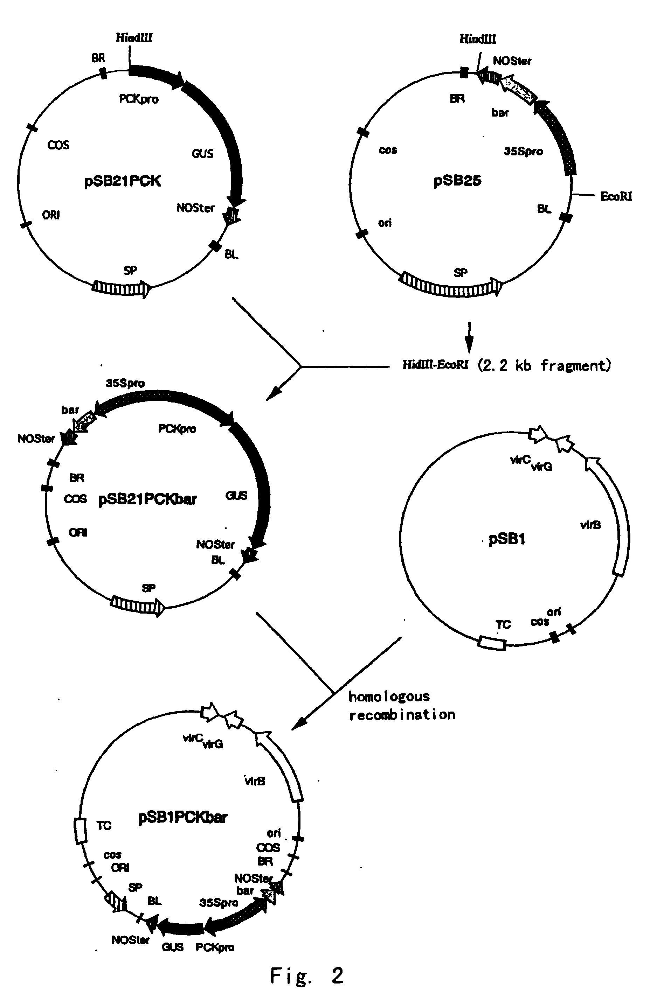

Each of the amplified fragment was replaced with the 35S promoter of an intermediate vector pSB21 for *Agrobacterium* at the BamHI and HindIII sites, which intermediate vector was prepared by removing the introns in the GUS gene of pSB24 (Komari T. et al, *supra*). The resulting vector was introduced into *E. coli* LE392 and then introduction into *Agrobacterium* and homologous recombination (Komari, T. et al., *supra*) were carried out by triple cross between *Agrobacterium* LBA4404/pSB4 (Komari T. et al., *supra*) and *E. coli* HB101/pRK2013 (Komari T. et al., *supra*). The resulting vectors were named pSB4PCK1 and pSB4PCK3, respectively. The structures of these recombinant vectors are shown in Fig. 1. In Fig. 1 and in Fig. 2 described below, both the PCK1 gene and PCK3 gene are designated "PCKpro". Further, in Figs. 1 and 2, each symbol has the following meaning:
- BL:: left border sequence of T-DNA of *Agrobacterium*
- BR:: right border sequence of T-DNA of *Agrobacterium*
- TC:: tetracycline resistant gene
- SP:: spectinomycin resistant gene
- HPT:: hygromycin resistant gene
- bar:: bar gene
- COS, cos:: COS site of λ phage
- ORI, ori:: replication origin
- 35Spro:: 35S promoter of cauliflower mosaic virus (CaMV)
- NOSter:: terminator of nopaline synthetase
- virB:: *vir*B gene of Ti plasmid pTiBo542 contained in *Agrobacterium tumefaciens* A281
- virC:: *vir*C gene of Ti plasmid pTiBo542 contained in *Agrobacterium tumefaciens* A281
- virG:: *vir*G gene of Ti plasmid pTiBo542 contained in *Agrobacterium tumefaciens* A281
- Vir:: entire *vir* regions of Ti plasmid of *Agrobacterium*
- GUS:: β-glucuronidase gene

### (3) Preparation of Transformed Rice by Agrobacterium method

Using the *Agrobacterium* into which the recombinant vector was introduced as mentioned above, and using Japonica rice variety "Tsukinohikari" as the plant to be transformed, transformation was performed by the *Agrobacterium* method according to the reported method (Hiei, Y. et al., (1994) Plant J. 6: 271-282). The obtained transformants were grown in an air-conditioned green house (16 hours daylight, day: 28°C, night: 23°C).

### (4) Identification of Expressing Tissue by GUS Staining and Measurement of GUS Activity of Green Leaves

Small sections of the leaves and roots of regenerated young plants were immersed in 50 mM phosphate buffer (pH 7) containing 20% methanol and 1 mM 4-methylumbelliferyl-β-D-glucuronide and incubated therein overnight at 37°C, thereby carrying out GUS staining. The plants which showed GUS expression were transplanted to pots. (As a negative control, one plant which did not show expression was also transplanted to a pot). The β-glucuronidase activity of the green leaves of the transformed plants grown in an air-conditioned green house (day: 28°C/night: 23°C, 16 hours daylight) was measured by the method of Matsuoka et al. (Makoto Matsuoka and Yasuharu Sanada (1991) Mol Gen Genet 225:411-419) with the fluorescent light of 4-methylumbelliferyl-β-D-glucuronide (4MU). The amount of the protein was measured by using BioRad Protein Assay (trademark, Japan BioRad Laboratories). Tissue-specific expression was checked for sections of green leaves, stems and roots, and for glumes by the method of Matsuoka et al. (Makoto Matsuoka and Yasuharu Sanada (1991) Mol Gen Genet 225: 411-419). The sections were prepared using a microslicer DTK-2000 (Produced by Dohan E M) under the conditions called speed 2, frequency 7, and the GUS staining reaction was carried out overnight at 37°C.

### (5) Results

Green leaves of 6 GUS staining-positive plants among the transformed plants into which pSB4PCK1 was introduced, 5 GUS staining-positive plants among the transformed plants into which pSB4PCK3 was introduced, and of one plant which was GUS staining-negative were checked for the GUS activities. As a result, plants showing high GUS activities were obtained for both transformants (Table 2). Sections of green leaves, stems and of roots, as well as glumes were checked forte expression of GUS, and strong expression was observed in the vicinity of sieve tubes from the leaves to the roots, but expression was not observed in other tissues containing photosynthesis cells. Expression was also observed in the hairs on the surfaces of glumes, pollen and anthers (Table 3). Clear difference was not observed in the tissue specificity of expression pattern between the transformed rice plants into which pSB4PCK1 and pSB4PCK3 were introduced, respectively, although the intensities of staining were different.

Based on the above-described results, it was thought that the gene fragments of PCK1 and PCK3 of *Urochloa panicoides* have promoter activities which express genes specifically in the vicinity of sieve tubes (from roots to leaves through stems) of C3 plants (at least rice). Further, it was confirmed that the gene fragments have promoter activities which express genes in glumes, pollen and anthers.

**Table 2**

| GUS Activity in Green Leaves (pmol/min/mg protein) | | | |
|---|---|---|---|
| | PCK1 | PCK3 | Control |
| 1 | 1013 | 39750 | 12.4 |
| 2 | 934 | 6476 | |
| 3 | 2053 | 261 | |
| 4 | 8849 | 597 | |
| 5 | 380 | 5152 | |
| 6 | 2952 | | |

**Table 3**

| Tissues Which Showed GUS Expression | |
|---|---|
| Leaf Blade | Cells in large and small vascular bundles (especially the region sandwiched between sieve tubes and vessels) |
| Leaf Sheath | Cells in large and small vascular bundles (especially the region sandwiched between sieve tubes and vessels) |
| Stem | Nodal net vascular bundles, circumferencial vascular bundle ring, root rudiment |
| Root | Cells in large and small vascular bundles |
| Ear | Surfaces and hairs of lemma and palet, rachis-branch |
| Anther | Filament vascular bundles, pollen |

### Example 2

### (1) Construction of Vector for Maize

The 35S-bar-nos fragment with a size of 2.2 kb which was cut out from pSB25 (described in WO 95/06722) by digestion with HindIII and EcoRI was inserted into the HindIII site of pSB21PCK which was constructed for rice. The obtained vector was introduced into *E. coli* LE392, and introduction to *Agrobacterium* and the homologous recombination (Komari, T. et al., Plant J. (1996) 10 : 165-174) were carried out by triple cross between *Agrobacterium* LBA4404/pSB1 and *E. coli* HB101/pRK2013 (Fig. 2). The resulting vectors were named pSB4PCK1bar and pSB4PCK3bar.

### (2) Transformation of Maize

Transformation of maize was carried out in accordance with the reported method (Y.Ishida, S.Saito, S.Ohta, Y.Hiei, T. Komari and T. Kumashiro (1996) High efficiency transformation of maize (Zea mays L.) mediated by Agrobacterium tumefaciens. Nature Biotechnology 14:745-750) using *Agrobacterium*.

### (3) Identification of Expressing tissue by GUS Staining and Measurement of GUS Activity

GUS staining of green leaves and roots of transformed maize were carried out by immersing them in 50 mM phosphate buffer (pH 7) containing 20% methanol and 1 mM 4-methylumbelliferyl-β-D-glucuronide overnight at 37°C. To check the cell-specific expression, sections were prepared using a microslicer DTK-2000 (produced by Dohan E M) under the conditions called speed 2, frequency 7. The β-glucuronidase activity was measured in accordance with the method by Matsuoka et al. (Makoto Matsuoka and Yasuharu Sanada (1991) Mol Gen Genet 225: 411-419) with the fluorescence of 4-methylumbelliferyl-β -D-glucuronide (4MU). The amount of the protein was measured by using BioRad Protein Assay (trademark, Japan BioRad Laboratories).

### (5) Results

Plants showing GUS activities in green leaves and roots were obtained from both transformants to which pSB1PCK1bar and pSB1PCK3bar were introduced, respectively. Tissues were subjected to GUS staining, and specific expression in green leaves and in the vascular bundles in roots was observed. Clear difference was not observed in the expression sites between the PCK1 and PCK3 promoters. These results were similar to those obtained for rice.

These results prove that PCK1 and PCK3 show promoter activities specifically in the vascular bundles of not only C3 plants, but also of C4 plants.

**Table 4**

| GUS Activity (pmol/min/mg protein) of Present Generation (R0) of Transformants | | | | |
|---|---|---|---|---|
| No. | PCK1 | | PCK3 | |
| | Green Leaf | Root | Green Leaf | Root |
| 1 | 132 | 60 | 958 | 121 |
| 2 | 2220 | 206 | 1170 | 122 |
| 3 | 699 | 41 | 173 | 97 |
| 4 | 530 | 119 | 376 | 74 |
| 5 | 2010 | 170 | 173 | 140 |
| 6 | 1270 | 85 | 458 | 107 |
| 7 | 10201 | 1225 | 386 | 84 |
| 8 | 4004 | 1021 | 3471 | 227 |
| 9 | 669 | ND | 1602 | ND |
| 10 | 5605 | ND | | |
| 11 | 1933 | ND | | |
| 12 | 1290 | ND | | |
| 13 | 4814 | ND | | |
| 14 | 994 | ND | | |
| 15 | 3040 | ND | | |
| 16 | 10859 | ND | | |
| 17 | 360 | ND | | |
| 18 | 167 | ND | | |
| 19 | 2834 | ND | | |
| 20 | 5111 | ND | | |
| 21 | 9848 | ND | | |
| ND: not determined | | | | |

## Claims

1. A nucleic acid having the nucleotide sequence shown in SEQ ID NO: 1 in SEQUENCE LISTING or a nucleic acid having a nucleotide sequence which is the same as the nucleotide sequence shown in SEQ ID NO: 1 in SEQUENCE LISTING except that one or a plurality of nucleotides are substituted, deleted, inserted or added, the latter nucleic acid having a promoter activity in phloem tissue of a plant, or a nucleic acid which is a part of anyone of said nucleic acids, that has a promoter activity in phloem tissue of a plant.

2. The nucleic acid according to claim 1, which hybridizes with the nucleic acid having the nucleotide sequence shown in SEQ ID NO: 1 in SEQUENCE LISTING under stringent conditions.

3. The nucleic acid according to claim 1, of which nucleotide sequence has a homology of not less than 70% with the nucleotide sequence shown in SEQ ID NO: 1 in SEQUENCE LISTING.

4. The nucleic acid according to claim 1, which is the nucleic acid having the nucleotide sequence shown in SEQ ID NO:. 1 in SEQUENCE LISTING or a part thereof which has a promoter activity in phloem tissue of a plant.

5. A nucleic acid having the nucleotide sequence shown in SEQ ID NO: 2 in SEQUENCE LISTING or a nucleic acid having a nucleotide sequence which is the same as the nucleotide sequence shown in SEQ ID NO: 2 in SEQUENCE LISTING except that one or a plurality of nucleotides are substituted, deleted, inserted or added, the latter nucleic acid having a promoter activity in phloem tissue of a plant, or a nucleic acid which is a part of anyone of said nucleic acids, that has a promoter activity in phloem tissue of a plant.

6. The nucleic acid according to claim 5, which hybridizes with the nucleic acid having the nucleotide sequence shown in SEQ ID NO: 2 in SEQUENCE LISTING under stringent conditions.

7. The nucleic acid according to claim 5, of which nucleotide sequence has a homology of not less than 70% with the nucleotide sequence shown in SEQ ID NO: 2 in SEQUENCE LISTING.

8. The nucleic acid according to claim 5, which is the nucleic acid having the nucleotide sequence shown in SEQ ID NO: 2 in SEQUENCE LISTING or a part thereof which has a promoter activity in phloem tissue of a plant.

9. A recombinant vector comprising the nucleic acid according to any one of claims 1 to 8.

10. The recombinant vector according to claim 9, further comprising a desired structural gene which is functionally ligated to a site downstream of said nucleic acid and which is controlled by said nucleic acid as a promoter.

11. A transformed plant transformed with said nucleic acid according to any one of claims 1 to 8 and a desired structural gene which is functionally ligated to a site downstream of said nucleic acid and which is controlled by said nucleic acid as a promoter, which transformed plant expresses said structural gene in its phloem tissue.

12. The transformed plant according to claim 11, which belongs to the family Gramineae.

13. The transformed plant according to claim 12, which is rice or maize.
